# EUROPEAN PATENT SPECIFICATION

(11) **EP 1 809 664 B1**
(45) Date of publication and mention of the grant of the patent: **14.05.2008**
(21) Application number: 04821383.9
(22) Date of filing: 09.11.2004
(51) Int. Cl.: C07K 14/61, C07K 1/20

(54) **A NOVEL PROCESS FOR PURIFICATION OF HUMAN GROWTH HARMONE**
NEUES VERFAHREN ZUR AUFREINIGUNG VON HUMANEM WACHSTUMSHORMON
NOUVEAU PROCEDE POUR LA PURIFICATION D'UNE HORMONE DE CROISSANCE HUMAINE

(43) Date of publication of application: 25.07.2007
(73) Proprietor: USV Limited, Mumbai 400088, Maharashtra (IN)
(72) Inventor: BORBHUIYA, Ahmed Monsur, Navi Mumbai 400 614 (IN); RAO, Yada Madhava, Khammam Andra Pradesh 507002 (IN); RAO, Laxmi Srinivas, Mumbai 400 008 (IN); MAITI, Dipanwita, Colaba, Mumbai 400 005 (IN); NIPHADKAR, Prabhakar Milind, Navi Mumbai 400 706 (IN); MISHRA, Shrikant, Mumbai 400 088 (IN)
(74) Representative: Henkel, Feiler & Hänzel
(86) International application number: PCT/IN2004/000346
(87) International publication number: WO 2006/051554

(56) References cited:
- EP-A- 0 321 940
- WO-A-00/52049
- WO-A-86/00339
- DD-A5- 285 113
- US-A- 4 332 717
- PAVLU BOHDAN ET AL: "Hydrophobic interaction chromatography of recombinant human growth hormone, genotropin" BIOSEPARATION, vol. 3, no. 5, 1992, pages 257-265, XP009049434 ISSN: 0923-179X cited in the application
- GELLERFORS P ET AL: "Separation and identification of growth hormone variants with high performance liquid chromatography techniques." ACTA PAEDIATRICA SCANDINAVICA. SUPPLEMENT. 1990, vol. 370, 1990, pages 93-100, XP009049430 ISSN: 0300-8843 cited in the application
- LEFORT S ET AL: "HYDROPHOBIC ADSORBENTS FOR THE ISOLATION AND PURIFICATION OF BIOSYNTHETIC HUMAN GROWTH HORMONE FROM CRUDE FERMENTATION MIXTURES" JOURNAL OF CHROMATOGRAPHY, vol. 361, 1986, pages 209-216, XP009049429
- WU S L ET AL: "Application of high-performance hydrophobic-interaction chromatography to the characterization of recombinant DNA-derived human growth hormone." JOURNAL OF CHROMATOGRAPHY. 2 FEB 1990, vol. 500, 2 February 1990 (1990-02-02), pages 595-606, XP002332906 ISSN: 0021-9673 cited in the application
- JACOBSON F S ET AL: "Role of high-performance liquid chromatographic protein analysis in developing fermentation processes for recombinant human growth hormone, relaxin, antibody fragments and lymphotoxin" JOURNAL OF CHROMATOGRAPHY A, ELSEVIER, AMSTERDAM, NL, vol. 763, no. 1, 28 February 1997 (1997-02-28), pages 31-48, XP004058714 ISSN: 0021-9673
- DE OLIVEIRA J E ET AL: "High-yield purification of biosynthetic human growth hormone secreted in Escherichia coli periplasmic space" JOURNAL OF CHROMATOGRAPHY A, ELSEVIER, AMSTERDAM, NL, vol. 852, no. 2, 13 August 1999 (1999-08-13), pages 441-450, XP004177405 ISSN: 0021-9673

## Description

### Technical field

The invention relates to the purification process for human growth hormone (hGH) in commercial scale by using hydrophobic interactive chromatography, which effectively separates clipped hormone moieties formed during the production of growth hormone by DNA recombinant techniques.

### Background of the invention

Human Growth Hormone (hGH) is a pituitary derived protein with a number of important biological functions, including protein synthesis, cell proliferation and metabolism. hGH is a 191 amino acid residue polypeptide of approximately 22 kDa. Recombinant human growth hormone (hGH) has been expressed in E.coli both as intracellular as well as secretary protein. A series of chromatographic and/or non-chromatographic methods are then used to obtain the pure protein. It is reported that the region between 140-150 amino acid residues in human growth hormone is sensitive to a number of proteases. This leads to a proteolytically cleaved form of protein whose physical properties are indistinguishable from that of intact molecule. This variant form of hGH can arise during the purification process and its removal poses a major challenge in the production of therapeutic grade growth hormone protein. To enable the use of hGH for therapeutic purpose, it is necessary to remove the clipped molecules.

Prior art describes techniques, which are different from the present invention in many aspects such as process parameters, equipments used, priority of separation of target moiety, use of number of phases, use of solvents, type of impurity separated. A few prior art documents disclose techniques, which are more useful as analytical techniques rather than as industrial processes of separation.

US 4332717 disclose the use of hydrophobic interaction chromatography for purifying human growth hormone. This patent purifies hGH extracted from the human pituitary glands and is not related to recombinant hGH or its purification. Hence, the impurities arising in this process are different from the one present in the recombinant hormone. Besides use of different media and different columns, the process uses different pressure conditions, different temperature conditions and different binding and elution buffer. The elution gradients are also different. It also describes the use of blue sepharose or agarose in separation and does not teach use of organic and aqueous mixture for elution, which is one of the essential features of the present invention.

Following references relate to purification of growth hormone obtained by recombinant DNA techniques. In the literature, there are known a few patents like US4861868, which describes the method for producing a recombinant porcine Growth Hormone; US4705848, US4694073, US4731440, US5064943, US4975529, US5023323, US5109117 and US6410694 deal with solubilization and naturation methods as an essential feature, but these are outside the scope of the present invention.

US patent 6022858 is related to the formulation of hGH wherein the hGH is pre- treated with Zn and optionally with lysine or calcium ions, after which benzyl alcohol is added to it and the pH adjusted to 2-9.

US patent 5734024 teaches a method for determination of biological activity of recombinant hGH and is good for analytical purposes.

US patent 5182369 teaches a method where the operations are carried out at a pH less than 6.5 and two-step precipitation is the essential feature. However, process conditions that employ acidic pH may lead to aggregation or acid hydrolysis of proteins and are a disadvantage.

US patent 6451347 describes a purification method for hGH, wherein complexation with metal ions such as Zn ions is carried out which is not a feature of the present invention. This patent describes the variants arising from the degradation of hGH and not the clipped moieties.

US patent 6451987 emphasizes the use of cation exchanger for purification of peptides including hGH, but does not mention the separation of the clipped hormone moiety from the intact hGH molecule

US patent 6437101 teaches a technique wherein aqueous biphasic extraction without the use of chaotropic agents is employed. The process is cumbersome and hence not easy to perform.

Even today the real problem in such purification is separation of clipped molecules resulting from mega target molecule. These clipped moieties remain bundled together due to certain other linkages present in the molecule.

Non-patent prior art documents comprises of following three published papers on use of hydrophobic interaction chromatography for separation of hGH variants, including clipped variant.

Pavlu and Gellerfors, Bioseparation (1993), 3: 257-265 disclose hydrophobic interaction chromatography of a recombinant human growth hormone, Genotropin.

Gellerfors et al., Acta Pediatr Scand (Suppl) (1990), 370: 93-100 teaches separation and identification of growth hormone variants by high performance liquid chromatography techniques.

Wu et al., J. Chromatogr. (1990), 500: 595-606 disclose Application of high-performance hydrophobic interaction chromatography to the characterization of DNA derived human growth hormone.

The techniques disclosed in all these references make use of a surfactant Brij (polyoxyethylene 23 lauryl ether) in mobile phase B and peaks corresponding to clipped molecule appear after the main peak .These papers disclose use of acetonitrile percentage of 0.5% in mobile phase A and 5% in mobile phase B along with 0.075% Brij. Brij 35 was used to improve recovery of the product from 70% to 99%. Brij 35, being a detergent helps in lowering the interaction of the protein with the matrix thereby and improving recovery. Interestingly, the peak of target molecule precedes the peak of variants. These papers essentially describe HPLC techniques and are better used for analytical purposes rather than for industrial production purposes. The main drawback of these methods is that the use of detergents like Brij is not favoured in industrial scale since its removal from the protein containing solution may be difficult.

The present invention makes use of organic solvents in much higher proportions and eliminates the use of Brij. Further it is noticed that the peak corresponding to variant precedes the peak of target molecule.

Prior art mentions resolution between clipped and intact molecules at analytical level loadings of approximately 50-150 µg semi-purified protein per 3.3 ml column volume. In the present invention resolution is achieved at a loading of 1 mg semi-purified protein per ml column volume.

The limitation of the prior art is the use of a non-ionic surfactant, Brij 35 to enhance the process recovery at an analytical level. This presence of non-ionic surfactant may be undesirable at preparative level, as it is difficult to remove surfactants that remain bound to protein molecules. The claimed process circumvents the need to use surfactant for improving process recovery by using higher amounts of organic solvent in the eluant that can be easily removed by tangential flow filtration or gel filtration chromatography.

### Objects of the invention

The main object of the invention is to describe a process for purification of human growth hormone that can effectively separate the clipped molecules from the target molecule.

Another object of the invention is to provide a process for purification of human growth hormone that effectively removes the clipped molecules first thereby enabling better control over production of target molecule.

Yet another objective of the present invention is to describe a process for purification of human growth hormone that can be used for multiple purposes such as analytical method for hGH, isolation of the hGH as well as industrial purification of hGH.

Still another objective of the invention to provide a purification process that can be effectively carried out in the pH range of 8.0 to 9.0.

Still yet another objective of the present invention is to develop a process for purification of human growth hormone that does not make use of any detergents or surfactants.

### Summary of the Invention

The invention relates to a novel process for purification of hGH obtained by recombinant technique using hydrophobic interactive chromatography. The invention further relates to the use of polymeric hydrophobic beads as solid support and mixture of aqueous buffers and organic solvents as an eluant to separate target hGH molecule from clipped hormone moieties present as impurities and finally desalting by gel filtration and lyophilizing to obtain purified hGH.

The features of the present invention will become more apparent from the following description of the inventive concept and the description of the referred embodiments.

### Brief description of the accompanying Figures

The following table describes chromatographic separation conditions for semi-purified hGH with respect to each figure.

| Figure # | Chromatography | Buffer A | Buffer B | Sample details |
|---|---|---|---|---|
| 1 | Column: Resource PHE (1.0 ml) Amersham | 20 mM Sod-Phosphate/ 0.4M K₂HPO₄, **pH 9.0** | 20 mM Sod-Phosphate, **pH 9.0** / **50 % Acetonitrile** | Sample: 1.0 mg Detection 280 nm |
| 2 | Column: Resource PHE (1.0 ml) Amersham | 20 mM Sod-Phosphate/ 0.4 M K₂HPO₄, **pH 9.0** | 20 mM Sod-Phosphate, **pH 9.0** / **40 % Acetonitrile** | Sample volume: 1.0 ml Detection 280 nm |
| 3 | Column: Resource PHE (1.0 ml) Amersham | 20 mM Sod-Phosphate/ 0.4 M K₂HPO₄, **pH 9.0** | 20 mM Sod-Phosphate, **pH 9.0** / **30 % Acetonitrile** | Sample volume: 1.0 ml Detection 280 nm |
| 4 | Column: Resource PHE (1.0 ml) Amersham | 20 mM Sod-Phosphate/ 0.4M K₂HPO₄, **pH 8.0** | 20 mM Sod-Phosphate, **pH 8.0** / 50 % Acetonitrile | Sample 1 mg Detection 280 nm |
| 5 | Column: Resource PHE (1.0 ml) Amersham | 20 mM Sod-Phosphate / 0.4 M K₂HPO₄, **pH 7.0** | 20 mM Sod-Phosphate, **pH 7.0** / **50 % Acetonitrile** | Sample 1.0 mg Detection 280 nm |
| 6 | Column Resource PHE (1.0 ml) Amersham | 20 mM Sod-Phosphate / 0.4 M K₂HPO₄, **pH** 6.0 | 20 mM Sod-Phosphate, **pH 6.0** / 50 % Acetonitrile | Sample 1.0 mg Detection 280 nm |
| 7 | Column: Resource PHE (1.0 ml) Amersham | 20 mM Sod-Phosphate/ 0.4 M K₂HPO₄, **pH 9.0** | 20 mM Sod-Phosphate, **pH 9.0** / **50 % Methanol** | Sample: 1.0 mg Detection 280 nm |
| 8 | Column: Resource PHE (1.0 ml) Amersham | 20 mM Sod-Phosphate/ 0.4 M K₂HPO₄, **pH 9.0** | 20 mM Sod-Phosphate, pH **9.0** / 40 % Methanol | Sample: 1.0 mg Detection 280 nm |
| 9 | Column: Resource PHE (1.0 ml) Amersham | 20 mM Sod-Phosphate/ 0.4 M K₂HPO₄, **pH 9.0** | 20 mM Sod-Phosphate, **pH 9.0** / **30 % Methanol** | Sample 1.0 mg Detection 280 nm |
| 10 | Column: Resource Phenyl (1.0 ml) Amersham | 20 mM Sodium Phosphate/ 0.4 M K₂HPO₄, **pH 8.0** | 20 mM Sodium Phosphate, **pH 8.0/ 50 % Methanol** | Sample: 1 mg Detection: 280 nm |
| 11 | Column : Resource Phenyl (1.0 ml) Amersham | 20 mM Sodium Phosphate/ 0.4 M K₂HPO₄, **pH 7.0** | 20 mM Sodium Phosphate, **pH 7.0/ 50 % Methanol** | Sample: 1 mg Detection: 280 nm |
| 12 | Column : Resource Phenyl (1.0 ml) Amersham | 20 mM Sodium Phosphate/ 0.4 M K₂HPO₄, **pH 6.0** | 20 mM Sodium Phosphate, **pH 6.0/ 50 % Methanol** | Sample: 1 mg Detection: 280 nm |
| 13 | Column : Resource Phenyl (1.0 ml) Amersham | 20 mM Sodium Phosphate/ 0.4 M K₂HPO₄, **pH** 9.0 | 20 mM Sodium Phosphate, **pH** 9.0/ 50 % isopropanol | Sample: 1 mg Detection: 280 nm |
| 14 | Column : Resource Phenyl (1.0 ml) Amersham | 20 mM Sodium Phosphate/ 0.4 M K₂HPO₄, **pH** 9.0 | 20 mM Sodium Phosphate, **pH** 9.0/ 40 % isopropanol | Sample: 1 mg Detection: 280 nm |
| 15 | Column: Resource Phenyl (1.0 ml) Amersham | 20 mM Sodium Phosphate/ 0.4 M K₂HPO₄, **pH** 9.0 | 20 mM Sodium Phosphate, **pH** 9.0/ 30 % isopropanol | Sample: 1 mg Detection: 280 nm |
| 16 | Column: Resource Phenyl (1.0 ml) Amersham | 20 mM Sodium Phosphate/ 0.4 M K₂HPO₄, **pH** 8.0 | 20 mM Sodium Phosphate, **pH** 8.0/ 50 % isopropanol | Sample: 1 mg Detection: 280 nm |
| 17 | Column : Resource Phenyl (1.0 ml) Amersham | 20 mM Sodium Phosphate/ 0.4 M K₂HPO₄, **pH** 7.0 | 20 mM Sodium Phosphate, **pH** 7.0/ 50 % isopropanol | Sample: 1 mg Detection: 280 nm |
| 18 | Column : Resource Phenyl (1.0 ml) Amersham | 20 mM Sodium Phosphate/ 0.4 M K₂HPO₄, **pH** 6.0 | 20 mM Sodium Phosphate, **pH 6.0/** 50 % isopropanol | Sample: 1 mg of ion-exchange purified sample Detection: 280 nm |
| 19 | Column : Resource Phenyl (1.0 ml) Amersham | 20 mM Sodium Phosphate/ 0.4M K₂HPO₄, **pH** 9.0 | 20 mM Sodium Phosphate, **pH** 9.0/ 25 % Acetonitrile / 25% methanol | Sample: 1 mg Detection: 280 nm |
| 20 | Column : Resource Phenyl (1.0 ml) Amersham | 20 mM Sodium Phosphate/ 0.4 M K₂HPO₄, **pH** 9.0 | 20 mM Sodium Phosphate, **pH** 9.0/ 25 % Acetonitrile / 25% isopropanol | Sample: 1 mg Detection: 280 nm |
| 21 | Column: Resource Phenyl (1.0 ml) Amersham | 20 mM Sodium Phosphate/ 0.4 M K₂HPO₄, **pH** 9.0 | 20 mM Sodium Phosphate, **pH** 9.0/ 25% isopropanol / 25 % Methanol | Sample: 1 mg Detection: 280 nm |
| 22 | Column: Resource PHE (1.0 ml) Amersham | 20 mM Sod-Phosphate, **pH** 8.0 / 0.4 M K₂HPO₄ | 20 mM Sod-Phosphate, **pH** 8.0 50 % Acetonitrile | Sample: GH, IEX / fractions, Sample volume: 1.0 ml, Detection 280 nm |
| 23 | Column: Resource PHE (1.0 ml) Amersham | 20 mM Tris/ 0.4 M K₂HPO₄, **pH** 9.0 | 20 mM Tris, **pH** 9.0 / 50 % Acetonitrile | Sample: 1.0 mg Detection 280 nm |
| 24 | Column: Sephacryl S-200 HR (Amersham) 100 ml) bed volume | 9 mM sodium phosphate, **pH** 8.0 | | Detection: 280 nm |
| 25 | Column: Resource PHE (1.0 ml) Amersham | 20 mM Sod-Phosphate / 0.4 M K₂HPO₄, **pH** 8.0 | 20 M Na-P, **pH** 8.0 | Sample: 1 mg, detection 280 nm |
| 26 | Column: Phenyl Sepharose FF (1.0 ml), Amersham | 20 mM Sod-Phosphate / 0.4 M K₂HPO₄, **pH** 8.0 | 20 M Na-P/ 50 % acetonitrile, **pH** 8.0 | Sample: 1 mg, Detection 280 nm |
| 27 | Column; Source 15 Q, Amersham | 10 mM NH4Cl/10 mM Tris, **pH** 8.0 / 3 % EtOH | 20 mM NH₄Cl, **pH** 8.0 / 7.5 % EtOH / 500 mM NaCl | Sample: Cu-IDA purified hGH having clipped molecule Detection: 280 nm Fractions: 1 ml System used: FPLC at 1 ml /min flow rate Analyses: 10 µl of each fraction analysed on a SDS-PAGE gel followed by silver staining |
| 28 | Column: Superdex 75 HR 10/30, Amersham | 20 mM Tris, **pH** 8.0 / 5 % Glycerol / 150 mM NaCl | | Sample: hGH having clipped molecule - denatured in 6 M urea and reduced with 50 mM DTT for 2 hrs at RT |
| | | | | Detection: 280 nm Fractions: 1 ml System used: FPLC at 0.5 ml /min flow rate **Analyses:** 10 µl of each fraction analysed on a SDS-PAGE gel followed by silver staining |
| 29 | Column: HiTrap 1 ml columns of Phenyl Sepharose FF (high sub) / Butyl Sepharose FF / Phenyl Sepharose HP / Octyl Sepharose FF | 20 mM Sodium phosphate, **pH** 7.0 / 0.5 m (NH₄)₂SO₄ | 20 mM Sodium phosphate, **pH** 7.0 | Sample: hGH having clipped molecule Detection : 280 nm Fractions: 1 ml System used: AKTA Explorer at 1 ml /min flow rate |

### Detailed description of the invention

In accordance with the object, the present invention discloses a purification technique for hGH. *E. coli* cells containing recombinant human growth hormone (hGH) gene were grown under standard conditions in a 1 L shake flask. After 8-10 hrs of induction, cells were harvested by centrifuging for 15 min at 4 - 8°C. The supernatant was discarded and the cell pellet was suspended in Lysis buffer containing 20 - 50 mM Tris, pH 7.0 - 9.0 and 100 - 500 mM NaCl. Cells were disrupted using ultrasonication for 30 min. Temperature during disruption was maintained at 4 to 8°C by keeping the samples on ice.

The crude lysate was clarified by centrifugation at 16,000 rpm for 1 hr at 4°C. After centrifugation the pellet was discarded and to the clear supernatant, imidazole was added to give a final concentration of 20 - 40 mM. This was loaded onto a 10 ml column of Chelating sepharose beads charges with NiSO₄. The column was equilibrated at a flow rate of 5-20 ml/ min with buffer containing 20 - 50 mM Tris pH 7.0 - 9.0, 100 - 500 mM NaCl, 20 - 40 mM Imidazole. Unbound material washed away using the equilibration buffer and after the absorbance at 280 dropped to baseline, elution of bound proteins was carried out using elution buffer containing 20 - 50 mM Tris pH 7.0 - 9.0, 100 - 500 mM NaCl, 200 - 500 mM Imidazole. Protein concentration in the elution was measured, made to 5 - 10 mg/ml concentration and kept for enzymatic digestion at 4 - 10°C. Digestion was carried out for 15 - 24 hrs and stropped by adding 2 M solution of K₂HPO₄, pH 7-9.0 to a give a final concentration of 0.2 - 0,4M. The digested sample was loaded onto a Phenyl Sepharose FF column (column vol. = 15 ml) equilibrated with buffer containing 20 - 50 mM Tris, 0.2 - 0.4 M K₂HPO₄, pH 7.0 - 9.0 at a flow rate of 5-10 ml /min. Bound protein eluted by a linear gradient or aqueous disodium hydrogen phosphate or Tris buffer and organic solvent. This was loaded onto Q Sepharose FF column (column vol = 10 ml) equilibrated with 20 - 50 mM Tris pH 7.0 - 9.0. The elution was done using a 15 - 30 column volume linear gradient of 0% A to 3.0% (v/v) of buffer "A" containing 0.5 - 2M NaCl. The major peak at 280 nm containing human growth hormone was collected and a solution of 2 M K₂HPO₄ was added to achieve a final concentration of 0.2 0.6M.

This sample was analyzed varying pH, equilibrating buffers and eluants conditions using Hydrophobic Interaction Chromatography.

According to the invention, there is provided a process for the purification of human growth hormone from its clipped moieties of hGH molecule, by using hydrophobic interaction chromatography technique, the said process comprising steps of:
a. loading the sample on the column in presence of inorganic salt having concentration in the range of 0.2 - 0.6 M,
b. equilibrating the column of step (a) loaded with sample with an aqueous buffer having pH in the range of 6.0-9.0,
c. eluting the equilibrated column of step (b) with the linear gradient of an aqueous buffer and organic solvent in the range of 40 - 70% v/v having pH in the range of 8.0 - 9.0,
d. collecting and combining eluted fractions corresponding to hGH peak, concentrating the combined fractions,
e. desalting and lyophilizing the concentrated fractions of step(d) by filtering on Sephacryl S-200 gel equilibrated with disodium hydrogen phosphate solution of pH 6.0-9.0, and
f. obtaining purified human growth hormone.

The process utilizes semi purified human growth hormone as a sample for purification.

The column used is hydrophobic resin is a cross-linked polystyrene divinyl benzene polymer resin having attached hydrophobic ligand selected from a group consisting of ether, isopropyl, butyl, octyl and phenyl.

The hydrophobic ligand is preferably phenyl group.
The inorganic salt is selected from a group consisting of ammonium sulfate, disodium hydrogen phosphate, dipotassium hydrogen phosphate or sodium chloride, preferably disodium hydrogen phosphate and most preferably dipotassium hydrogen phosphate.

The inorganic salt concentration used is in the range of 0.2 - 0.6 M, more preferably 0.3 - 0.4 M.

The buffer used for equilibrating the column is a mixture of aqueous disodium hydrogen phosphate and dipotassium hydrogen phosphate. The pH of the equilibrating buffer ranges between 6.0 and 9.0, preferably in the range of 8.0 to 9.0.

The buffer used for eluting protein is a mixture of disodium hydrogen phosphate or tris buffer and an organic solvent.

The pH of eluting buffer preferably ranging between 8.0 and 9.0

The organic solvent used for eluting protein is selected from the group consisting of C₁ to C₄ alcohol, acetonitrile and mixtures thereof.

The organic solvent in the eluting mixture ranges between 40-70% v/v, more preferably 40% - 50% v/v.

The temperature for chromatography separations is preferably in the range of 20-30°C, more preferably 22-24°C.

The growth hormone peak eluted from the above hydrophobic interaction chromatography step was concentrated and further desalted on a Sephacryl S-200 gel filtration column equilibrated with 2 - 10 mM disodium hydrogen phosphate of pH 7.0 - 9.0. Desalted fraction collected and lyophilized to obtain pure hGH.

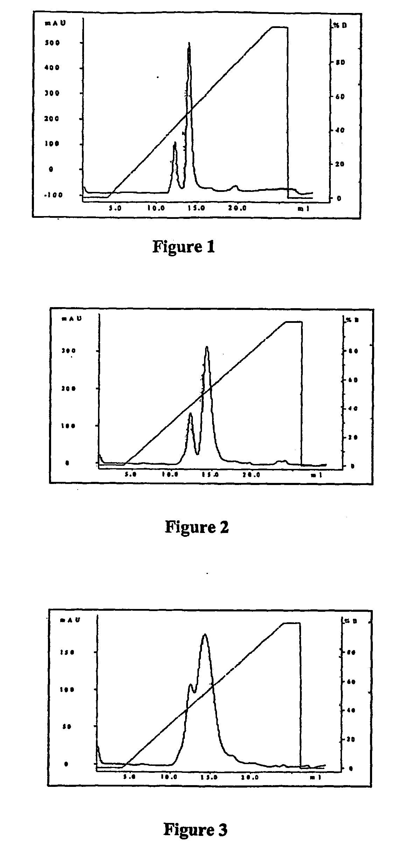

It is known that 0.5 to 5% acetonitrile cannot separate the clipped moieties from the recombinant hGH. (Gellerfors P et al. Acta Pediatr Scand (Suppl) (1990) 370, 93-100, Separation and identification of growth hormone variants by high performance liquid chromatography techniques In the present invention, it was found that use of acetonitrile in the range of 50%±10% effectively separates the unwanted clipped molecules first and subsequently the target molecule can be effectively separated with the purity of > 99.5 %. pH also plays an important role in the efficiency of separation. When the experiments were performed, it was observed that when the pH is acidic there was no effective separation whereas when the pH was increased above neutrality the separation became more and more effective. It was observed that pH range of 8 to 9 yields optimum purification and hence the desirable quality product (Figure 1, Figure 2). After conducting series of experiments it is concluded that pH does play an important role in achieving resolution between the clipped and intact hGH molecules using Resource Phenyl chromatography. Separation is most efficient at pH 8 - 9. Decreasing the pH below 8.0 reduces resolution (Figure 3) so much that at pH 6.0 both molecules elute as a single peak. In all the chromatograms the peak on the left corresponds to clipped hGH and on the right is of intact hGH.

Applicant tried various other techniques to separate clipped molecules from intact hGH were tried as mentioned herein below:
1. Ion-exchange with both polymer as well as sepharose beads- using aqueous buffer as well as mixture of aqueous-organic solvents and detergents
2. Hydrophobic interaction chromatography using sepharose beads of different hydrophobicities
3. Gel filtration chromatography uses native as well as reduced-denatured conditions

A brief account of the details of conditions used for purification and the outcome features below.

### Experimental details

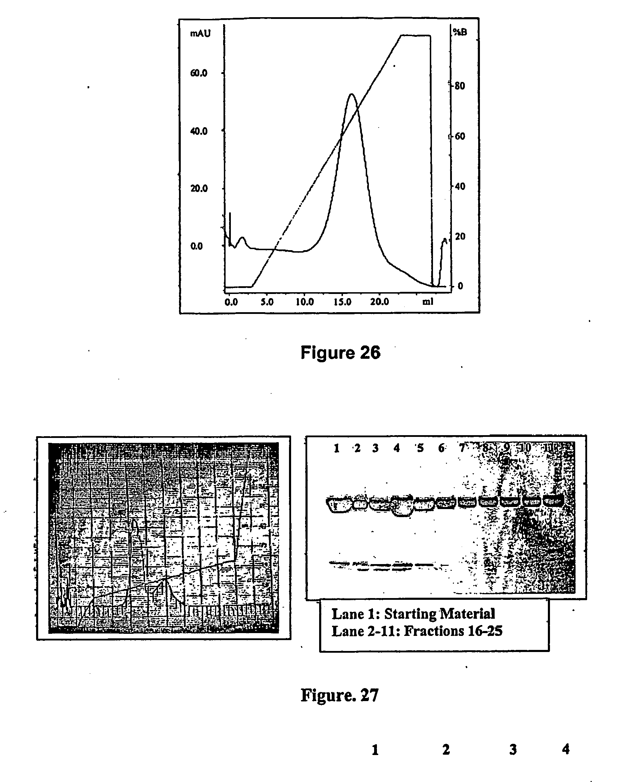

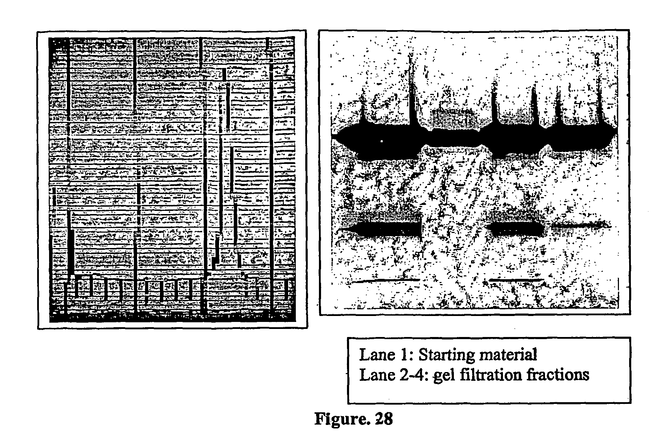

Ion-exchange chromatography: Ion exchange chromatography using Source 15 Q beads were tried to separate the intact from clipped hGH molecules using sodium chloride gradient for elution. (Figure 27)
**Observation:** No separation of Clipped molecules from intact hGH is observed in the above chromatographic process in any of the factions analysed by SDS-PAGE gel.
Gel filtration chromatography-
Column: Superdex 75 HR 10/30, Amersham
Buffer: 20 mM Tris, pH 8.0 / 5 % Glycerol / 150 mM NaCl
Sample: hGH having clipped molecule -denatured in 6 M urea and reduced with 50 mM DTT for 2 hrs at RT
Detection: 280 nm
Fractions: 1 ml
System used: FPLC at 0.5 ml /min flow rate
Analyses: 10 ul of each fraction analysed on a SDS-PAGE gel followed by silver staining (Figure 28)
**Observation:** No separation of Clipped molecules from intact hGH is observed in the above chromatographic process in any of the factions analysed by SDS-PAGE gel.

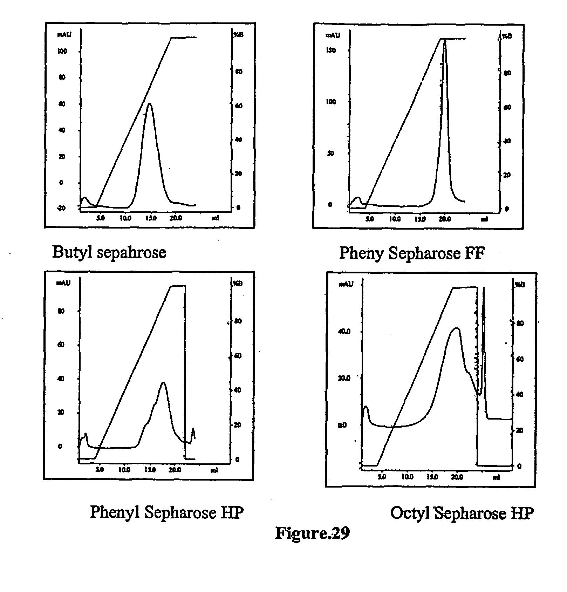

**Hydrophobic interaction Chromatography on Sepharose beads- (****Figure 29****)**
Column: HiTrap 1 ml columns of Phenyl Sepharose FF (high sub) / Butyl Sepharose FF / Phenyl Sepharose HP / Octyl Sepharose FF
Buffer A: 20 mM Sodium phosphate, pH 7.0 / 0.5 m (NH₄)₂SO₄
Buffer B: 20 mM Sodium phosphate, pH 7.0
Sample: hGH having clipped molecule
Detection: 280 nm
Fractions: 1 ml
System used: AKTA Explorer at 1 ml /min flow rate
**Observation:** No separation of Clipped molecules from intact hGH is observed in the above chromatographic process
The following examples are illustrative of the invention but not to be construed to limit the scope of the present invention. The present invention has been described in terms of its specific embodiments and certain modifications and equivalents will be apparent to those skilled in the art and are intended to be included within the scope of present invention.

### EXAMPLES

### Example 1

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 50 % acetonitrile. Fractions of 1 ml were collected and analysed by SDS-PAGE. The clipped hGH molecule eluted ahead of the intact GH molecules **(****Figure 1****).** Highly pure form of human growth hormone was obtained. (Purity : > 99.5%, Yield: 85%)

### Example 2

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 40 % acetonitrile. Fractions of 1 ml were collected and analysed by SDS-PAGE. The clipped hGH molecule eluted ahead of the intact GH molecules **(****Figure 2****).** Highly pure form of human growth hormone was obtained. (Purity: > 99.5%, Yield: 85%)

### Example 3

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 30 % acetonitrile. Fractions of 1 ml were collected and analysed by SDS-PAGE. The clipped hGH molecule did not resolve from intact molecule **(****Figure 3****)**.

### Example 4

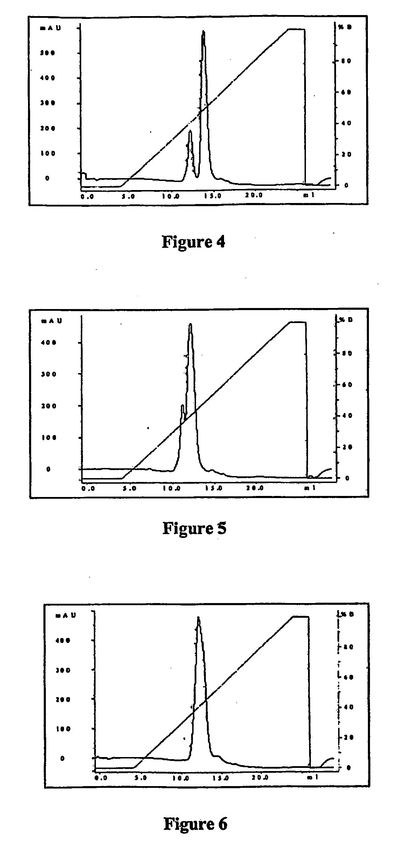

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 8.0. Bound proteins were eluted with a 20 ml linear gradient of to mM Na₂HPO₄, pH 8.0 / 50 % acetonitrile. Fractions of 1 ml were collected and analysed by SDS-PAGE. The clipped hGH molecule eluted ahead of the intact GH molecules **(****Figure 4****)**. Highly pure form of human growth hormone was obtained. (Purity: > 99.5%, Yield: 85%)

### Example 5

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 7.0. Bound proteins were eluted with a 20 ml linear gradient of 20 mM Na₂HPO₄, pH 7.0 / 50 % acetonitrile. Fractions of 1 ml were collected and analysed by SDS-PAGE **(****Figure 5****).** (Purity: >80%, Yield: 35%)

### Example 6

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 6.0. Bound proteins were eluted with a 20 ml linear gradient of 20 mM Na₂HPO₄, pH 6.0 / 50 % acetonitrile. Fractions of 1 ml were collected and analysed by SDS-PAGE. The clipped hGH molecule did not resolve from intact molecule **(****Figure 6****)**.

### Example 7

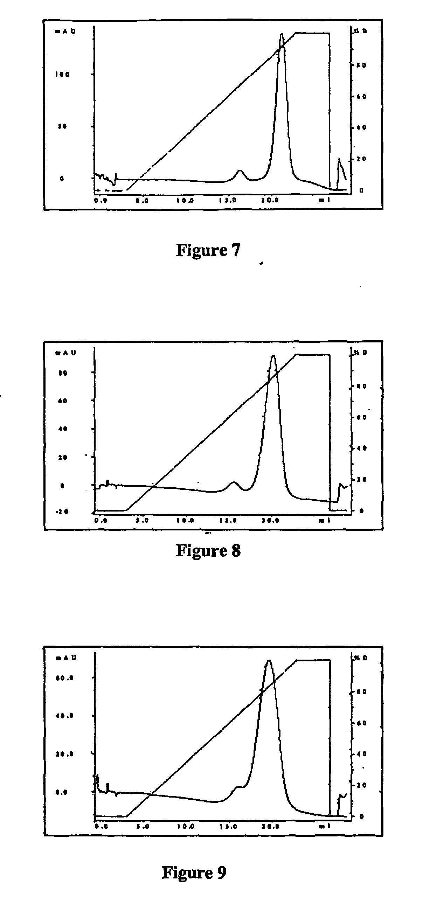

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 50 % Methanol. Fractions of 1 ml were collected and analysed by SDS-PAGE. The clipped hGH molecule eluted ahead of the intact GH molecules **(****Figure 7****)**. Highly pure form of human growth hormone was obtained. (Purity: > 99.5%, Yield: 85%)

### Example 8

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 40 % Methanol. Fractions of 1 ml were collected and analysed by SDS-PAGE. The clipped hGH molecule eluted ahead of the intact GH molecules **(****Figure 8****).** Highly pure form of human growth hormone was obtained. (Purity : > 99.5%, Yield: 85%)

### Example 9

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 30 % Methanol. Fractions of 1 ml were collected and analysed by SDS-PAGE. The clipped hGH molecule did not resolve from intact molecule **(****Figure 9****)**. (Purity : >95 %, Yield: 45%)

### Example 10

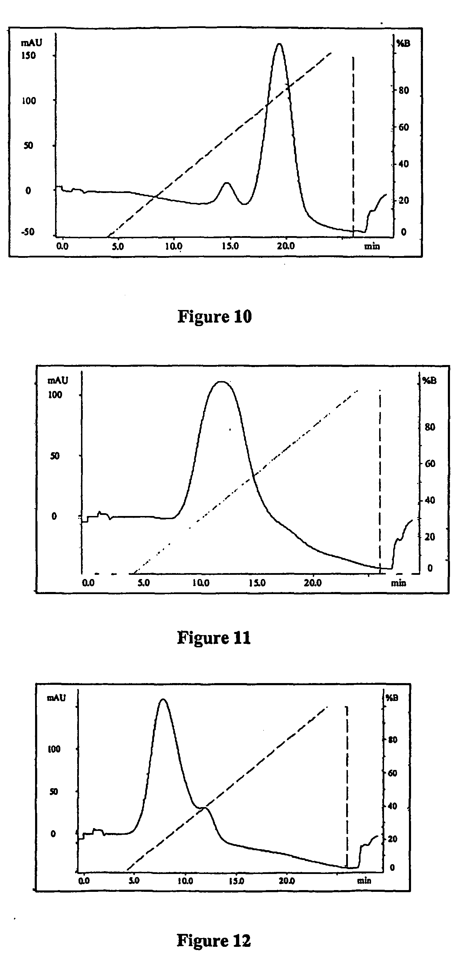

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 8.0. Bound proteins were eluted with a 20 ml linear gradient of 20 mM Na₂HPO₄, pH 8.0 / 50 % methanol. Fractions of 1 ml were collected and analysed by SDS-PAGE. The clipped hGH molecule eluted ahead of the intact GH molecules **(****Figure 10****)**. Highly pure form of human growth hormone was obtained. (Purity >98%; Yield 80%)

### Example 11

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 7.0. Bound proteins were eluted with a 20 ml linear gradient of 20 mM Na₂HPO₄, pH 7.0 / 50 % methanol. Fractions of 1 ml were collected and analysed by SDS-PAGE. The clipped hGH molecule eluted ahead of the intact GH molecules **(****Figure 11****).** (No purity achieved)

### Example 12

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄ / 0.4 M K₂HPO₄, pH 6.0. Bound proteins were eluted with a 20 ml linear gradient of 20 mM Na₂HPO₄, pH 6.0 / 50 % methanol. Fractions of 1 ml were collected and analysed by SDS-PAGE. The clipped hGH molecule eluted ahead of the intact GH molecules **(****Figure 12****).** (No purity achieved)

### Example 13

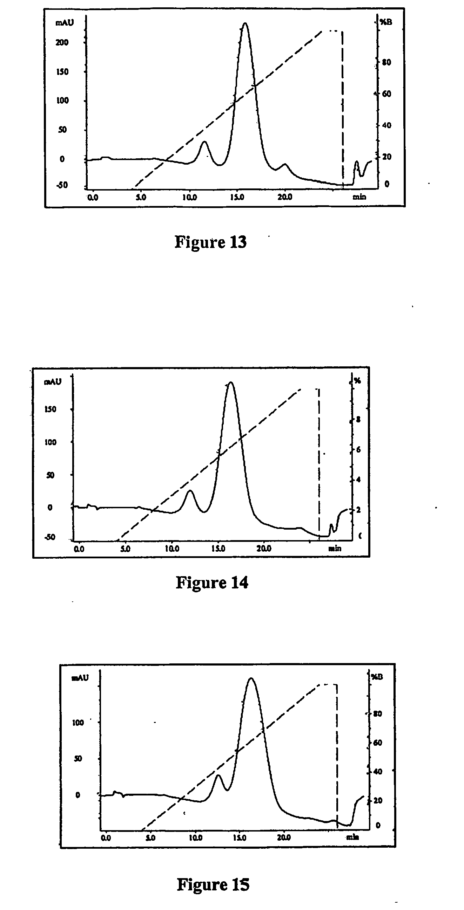

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 50 % isopropanol. Fractions of 1 ml were collected and analysed by SDS-PAGE. The clipped hGH molecule eluted ahead of the intact GH molecules **(****Figure 13****).** Highly pure form of human growth hormone was obtained (Purity > 99%; Yield 85%).

### Example 14

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 40 % isopropanol. Fractions of 1 ml were collected and analysed by SDS-PAGE. The clipped hGH molecule eluted ahead of the intact GH molecules **(****Figure 14****).** Highly pure form of human growth hormone was obtained (Purity >98%; Yield 80%).

### Example 15

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 30 % isopropanol. Fractions of 1 ml were collected and analysed by SDS-PAGE. The clipped hGH molecule eluted ahead of the intact GH molecules **(****Figure 15****)** (Purity >95%; Yield 60%).

### Example 16

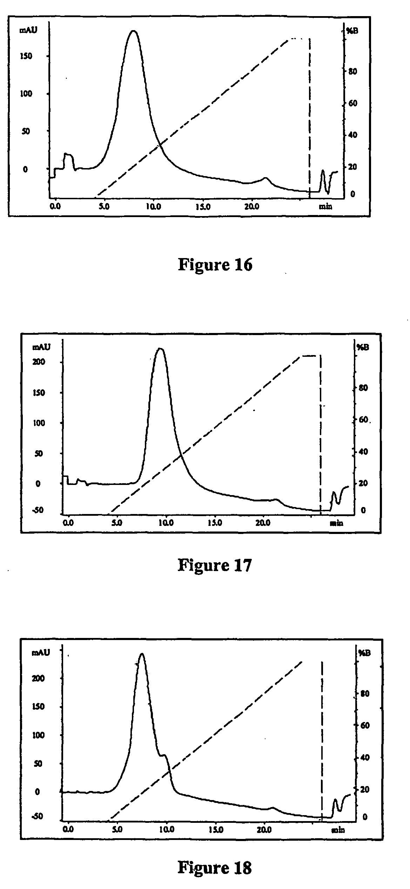

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 8.0. Bound proteins were eluted with a 20 ml linear gradient of 20 mM Na₂HPO₄, pH 8.0 / 50 % isopropanol. Fractions of 1 ml were collected and analysed by SDS-PAGE. The clipped hGH molecule eluted ahead of the intact GH molecules **(****Figure 16****)** (Purity not achieved).

### Example 17

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 7.0. Bound proteins were eluted with a 20 ml linear gradient of 20 mM Na₂HPO₄, pH 7.0 / 50 % isopropanol. Fractions of 1 ml were collected and analysed by SDS-PAGE. The clipped hGH molecule eluted ahead of the intact GH molecules **(****Figure 17****)** (no purity achieved).

### Example 18

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 6.0. Bound proteins were eluted with a 20 ml linear gradient of 20 mM Na₂HPO₄, pH 6.0 / 50 % isopropanol. Fractions of 1 ml were collected and analysed by SDS-PAGE. The clipped hGH molecule eluted ahead of the intact GH molecules **(****Figure 18****)** (no purity achieved).

### Example 19

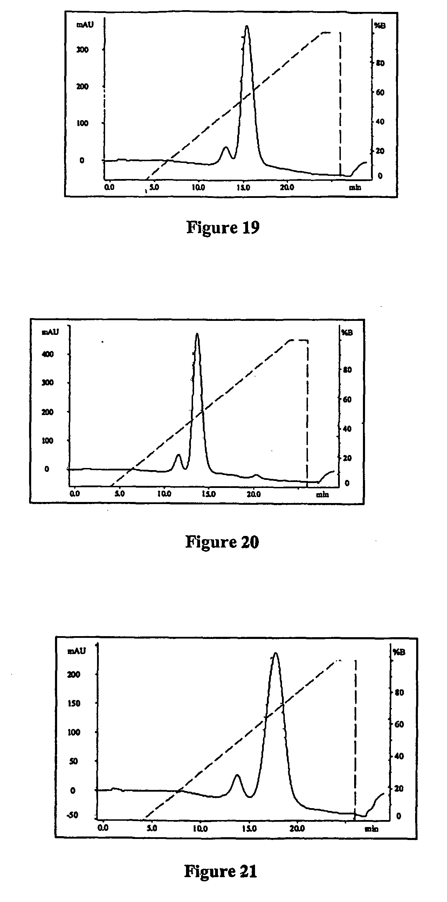

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 25 % acetonitrile / 25% methanol. Fractions of 1 ml were collected and analysed by SDS-PAGE. The clipped hGH molecule eluted ahead of the intact GH molecules **(****Figure 19****).** Highly pure form of human growth hormone was obtained (Purity >99%; Yield 80%).

### Example 20

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 25 % acetonitrile / 25% isopropanol. Fractions of 1 ml were collected and analysed by SDS-PAGE. The clipped hGH molecule eluted ahead of the intact GH molecules **(****Figure 20****).** Highly pure form of human growth hormone was obtained (Purity >99%; Yield 85%).

### Example 21

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 9.0. Bound proteins were eluted with a 20 ml linear gradient of 20 mM Na₂HPO₄, pH 9.0 / 25 % isopropanol / 25% methanol. Fractions of 1 ml were collected and analysed by SDS-PAGE. The clipped hGH molecule eluted ahead of the intact GH molecules **(****Figure 21****).** Highly pure form of human growth hormone was obtained (Purity >99%; Yield 85%).

### Example 22

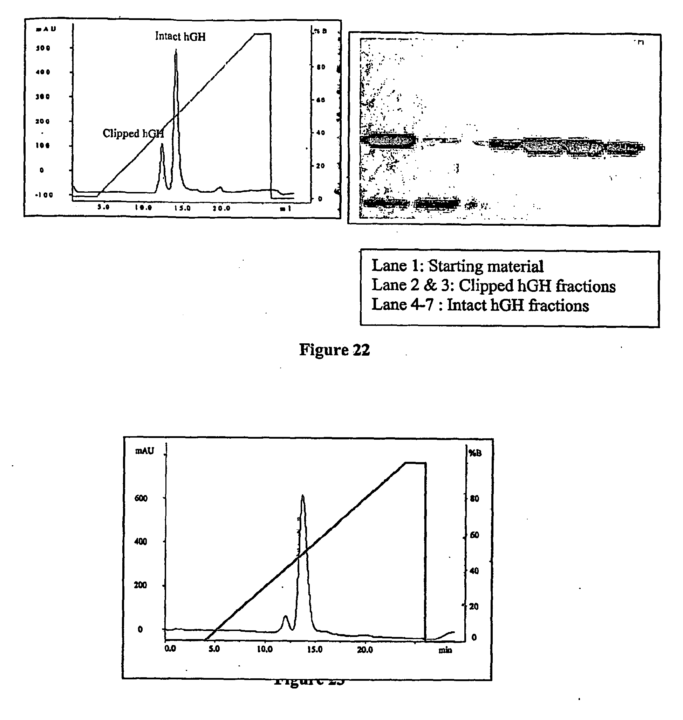

To the ion-exchange purified fraction of hGH, K₂HPO₄ is added to a final concentration of 0.4 M. This was injected onto a Resource Phenyl column (procured from Amersham Biosciences) equilibrated with 20 mM Na₂HPO₄/ 0.4 M K₂HPP₄, pH 9.0. Bound proteins were eluted with a 20 ml linear gradient of 20 mM Na₂HPO₄, pH 9.0 / 50 % acetonitrile. Fractions of 1 ml were collected and analysed by SDS-PAGE according to the method of Laemlli. The gel was run at 25 mA for 45 min and thereafter silver stained to visualize the protein bands **(****Figure 22****).** The clipped hGH molecule eluted ahead of the intact GH molecules and is resolved as seen from the gel picture.

### Example 23

To the ion-exchange purified fraction of hGH, K₂HPO₄ is added to a final concentration of 0.4 M. This was injected onto a 1 ml Resource Phenyl column (30 x 6.4 mm) equilibrated with 20 mM Tris/ 0.4 M K₂HPO₄, pH 9.0. Bound proteins were eluted with a 20 ml linear gradient of 20 mM Tris, pH 9.0 / 50 % acetonitrile. Fractions of 1 ml were collected and analysed by SDS-PAGE. The clipped hGH molecule eluted ahead of the intact GH molecules **(****Figure 23****).** Highly pure form of human growth hormone was obtained (Purity : > 99.5%, Yield: 85%).

### Example 24

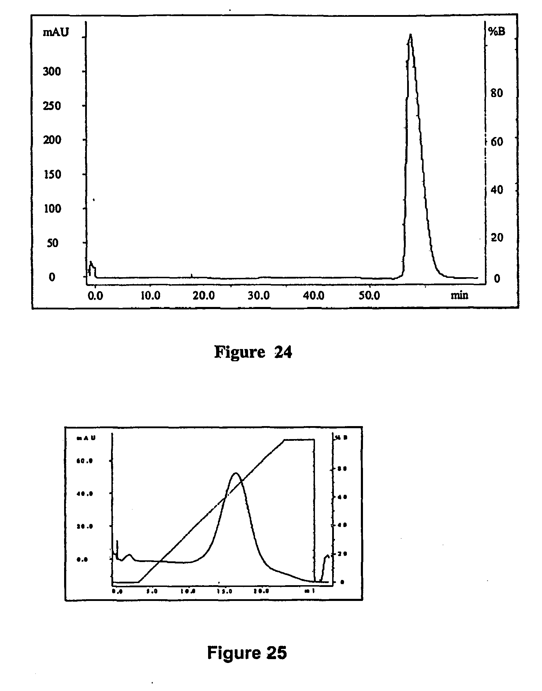

Resource PHE purified protein fraction was loaded to a 100 ml bed volume of Sephacryl S-200 HR column equilibrated with 9 mM disodium hydrogen phosphate buffer, pH 8.0. Column was eluted with the same buffer at 0.4 ml/min. Protein peak, detected at 280 nm was collected and lyophilised. Pure hGH (>99.5%) was obtained with a yield of >95% **(****Fig 24****).**

### Example 25

To the ion-exchange purified fraction of hGH, K₂HPO₄ is added to a final concentration of 0.4 M. This was injected onto a 1 ml Resource Phenyl column (30 x 6.4 mm) equilibrated with 20 mM Na₂HPO₄/ 0.4 M K₂HPO₄, pH 8.0. Bound proteins were eluted with a 20 ml linear gradient of 20 mM Na₂HPO₄, pH 8.0. Fractions of 1 ml were collected and analysed by SDS-PAGE. **(****Figure 25****).** No resolution of clipped molecules and intact hGH molecules are obtained.

### Example 26

To the ion-exchange purified fraction of hGH, K₂HPO₄ is added to a final concentration of 0.4 M. This was injected onto a 1 ml Phenyl Sepharose FF column equilibrated with 20 mM Na₂HPO₄/ 0.4 M K₂HPO₄, pH 8.0. Bound proteins were eluted with a 20 ml linear gradient of 20 mM Na₂HPO₄/ 50% acetonitrile, pH 8.0. Fractions of 1 ml were collected and analysed by SDS-PAGE. **(****Figure 26****).** No resolution of clipped molecules and intact hGH molecules are obtained.

## Claims

1. A process for the purification of human growth hormone from its clipped moieties or hGH molecule, by using hydrophobic interaction chromatography techniques, the said process comprising steps of:
a. loading the sample on the column in presence of inorganic salt having concentration in the range of 0.2 - 0.6 M,
b. equilibrating the column of step (a) loaded with sample with an aqueous buffer having pH in the range of 6.0-9.0,
c. eluting the equilibrated column of step (b) with the linear gradient of an aqueous buffer and organic solvent in the range of 40-70% v/v having pH in the range of 8.0-9.0,
d. collecting and combining the eluted fractions corresponding to hGH peak, concentrating the combined fractions,
e. desalting and lyophilizing the concentrated fractions of step (d) by filtering on Sephacryl S-200 gel equilibrated with disodium hydrogen phosphate solution of pH ranging between 6.0-9.0, and
f. obtaining purified human growth hormone.

2. A process as claimed in claim 1, wherein in step (a) the sample used is semi-purified human growth hormone.

3. A process of claim 1, wherein in step (a) the column used is hydrophobic resin comprising cross-linked polystyrene divinyl benzene polymer resin having attached hydrophobic ligand selected from the group consisting of ether, isopropyl, butyl, octyl and phenyl.

4. A process of claim 3, wherein the hydrophobic ligand is preferably phenyl.

5. A process of claim 1, wherein in step (a) the inorganic salt used is selected from a group consisting of ammonium sulfate, disodium hydrogen phosphate, dipotassium hydrogen phosphate or sodium chloride, preferably disodium hydrogen phosphate, and most preferably dipotassium hydrogen phosphate.

6. A process of claim 1, wherein in step (b) the aqueous buffer used for equilibrating the column is a mixture of aqueous disodium hydrogen phosphate and dipotassium hydrogen phosphate.

7. A process of claim 1, wherein in step (c) the aqueous buffer is either disodium hydrogen phosphate or Tris buffer.

8. A process of claim 1, wherein in the step (c) the organic solvent used is selected from the group consisting of C₁ to C₄ alcohol, acetonitrile and/or mixtures thereof.

9. A process of claim 1, wherein in step (c) the linear gradient range is preferably 40.50%.

10. A process of claim 1, wherein the temperature for chromatography separation is preferably in the range of 20-30°C, more preferably 22-24°C.

## Patentansprüche

1. Verfahren zur Reinigung von humanem Wachstumshormon von dessen gestutzten Einheiten des hGH-Moleküls unter Verwendung der Technik der hydrophoben Interaktionschromatographie, wobei das Verfahren die folgenden Stufen umfasst:
a. Laden der Probe auf die Säule in Gegenwart eines anorganischen Salzes mit einer Konzentration im Bereich von 0,2-0,6 M,
b. Äquilibrieren der mit der Probe beladenen Säule von Stufe (a) mit einem wässrigen Puffer mit einem pH-Wert im Bereich von 6,0-9,0,
c. Eluieren der äquilibrierten Säule von Stufe (b) mit einem linearen Gradienten eines wässrigen Puffers und eines organischen Lösemittels im Bereich von 40-70 % (V/V) mit einem pH-Wert im Bereich von 8,0-9,0,
d. Sammeln und Vereinigen der dem hGH-Peak entsprechenden eluierten Fraktionen, Konzentrieren der vereinigten Fraktionen,
e. Entsalzen und Lyophilisieren der konzentrierten Fraktionen von Stufe (d) durch Filtrieren auf einem Sephacryl S-200-Gel, das mit einer Dinatriumhydrogenphosphatlösung eines pH-Werts im Bereich zwischen 6,0 und 9,0 äquilibriert ist, und
f. Gewinnen von gereinigtem humanem Wachstumshormon.

2. Verfahren nach Anspruch 1, wobei die in Stufe (a) verwendete Probe halbgereinigtes humanes Wachstumshormon ist.

3. Verfahren nach Anspruch 1, wobei die in Stufe (a) verwendete Säule ein hydrophobes Harz ist, das ein vernetztes Polystyrol-divinylbenzol-polymerharz mit einem daran gebundenen hydrophoben Liganden, der aus der Gruppe von Ether, Isopropyl, Butyl, Octyl und Phenyl ausgewählt ist, umfasst.

4. Verfahren nach Anspruch 3, wobei der hydrophobe Ligand vorzugsweise Phenyl ist.

5. Verfahren nach Anspruch 1, wobei das in Stufe (a) verwendete anorganische Salz aus der Gruppe von Ammoniumsulfat, Dinatriumhydrogenphosphat, Dikaliumhydrogenphosphat oder Natriumchlorid, vorzugsweise Dinatriumhydrogenphosphat und noch besser Dikaliumhydrogenphosphat ausgewählt ist.

6. Verfahren nach Anspruch 1, wobei der in Stufe (b) zur Äquilibrierung der Säule verwendete wässrige Puffer ein Gemisch von wässrigem Dinatriumhydrogenphosphat und Dikaliumhydrogenphosphat ist.

7. Verfahren nach Anspruch 1, wobei der wässrige Puffer in Stufe (c) entweder Dinatriumhydrogenphosphat oder Tris-Puffer ist.

8. Verfahren nach Anspruch 1, wobei das in Stufe (c) verwendete organische Lösemittel aus der Gruppe von einem C₁- bis C₄-Alkohol, Acetonitril und/oder Gemischen derselben ausgewählt ist.

9. Verfahren nach Anspruch 1, wobei in Stufe (c) der Bereich des linearen Gradienten vorzugsweise 40-50 % beträgt.

10. Verfahren nach Anspruch 1, wobei die Temperatur zur chromatographischen Trennung vorzugsweise im Bereich von 20-30 °C, noch besser 22-24 °C liegt.

## Revendications

1. Procédé pour la purification d'une hormone de croissance humaine à partir de ses fragments de molécule de hGH tronqués, en utilisant une technique de chromatographie par interaction hydrophobe, ledit procédé comprenant les étapes consistant à :
a. charger l'échantillon sur la colonne en présence d'un sel inorganique ayant une concentration dans la gamme de 0,2 à 0,6 M,
b. équilibrer la colonne de l'étape (a) chargée avec l'échantillon avec un tampon aqueux ayant un pH dans la gamme de 6,0 à 9,0,
c. éluer la colonne équilibrée de l'étape (b) avec un gradient linéaire d'un tampon aqueux et d'un solvant organique dans la gamme de 40 à 70% v/v ayant un pH dans la gamme de 8,0 à 9,0,
d. récolter et combiner les fractions éluées correspondant au pic de hGH, à concentrer les fractions combinées,
e. dessaler et lyophiliser les fractions concentrées de l'étape (d) en filtrant sur du gel Sephacryl S-200 équilibré avec une solution d'hydrogénophosphate de disodium à pH allant entre 6,0-9,0, et
f. obtenir une hormone de croissance humaine purifiée.

2. Procédé selon la revendication 1, dans lequel, à l'étape (a), l'échantillon utilisé est une hormone de croissance humaine semi-purifiée.

3. Procédé selon la revendication 1, dans lequel, à l'étape (a), la colonne utilisée est une résine hydrophobe comprenant une résine de polymère polystyrène divinylbenzène réticulé ayant un ligand hydrophobe attaché choisi dans le groupe constitué par un éther, un isopropyle, un butyle, un octyle et un phényle.

4. Procédé selon la revendication 3, dans lequel le ligand hydrophobe est de préférence un phényle.

5. Procédé selon la revendication 1, dans lequel, à l'étape (a), le sel inorganique utilisé est choisi dans un groupe constitué par le sulfate d'ammonium, l'hydrogénophosphate de disodium, l'hydrogénophosphate de dipotassium ou le chlorure de sodium, de préférence l'hydrogénophosphate de disodium et de toute préférence l'hydrogénophosphate de dipotassium.

6. Procédé selon la revendication 1, dans lequel, à l'étape (b), le tampon aqueux utilisé pour équilibrer la colonne est un mélange d'hydrogénophosphate de disodium et d'hydrogénophosphate de dipotassium aqueux.

7. Procédé selon la revendication 1, dans lequel, à l'étape (c), le tampon aqueux est soit de l'hydrogénophosphate de disodium soit du tampon Tris.

8. Procédé selon la revendication 1, dans lequel, à l'étape (c), le solvant organique utilisé est choisi dans le groupe constitué par un alcool en C₁ à C₄, l'acétonitrile et/ou des mélanges de ceux-ci.

9. Procédé selon la revendication 1, dans lequel, à l'étape (c), la gamme de gradient linéaire est de préférence 40 à 50%.

10. Procédé selon la revendication 1, dans lequel la température pour la séparation chromatographique est de préférence dans la gamme de 20-30°C, de préférence encore 22-24°C.
